Europäisches Patentamt

European Patent Office

Office européen des brevets

(11) Publication number: **0 066 296 B1**

(12) **EUROPEAN PATENT SPECIFICATION**

(45) Date of publication of patent specification: **13.11.85**

(51) Int. Cl.⁴: **A 61 K 39/29**, G 01 N 33/576

(21) Application number: **82104830.3**

(22) Date of filing: **02.06.82**

(54) **Non-A, non-B hepatitis-associated antigen, a conjugate thereof and diagnostic reagent containing same.**

(30) Priority: **02.06.81 JP 83736/81**

(43) Date of publication of application:
**08.12.82 Bulletin 82/49**

(45) Publication of the grant of the patent:
**13.11.85 Bulletin 85/46**

(84) Designated Contracting States:
**BE CH DE FR GB IT LI NL SE**

(56) References cited:
**WO-A-80/02598**
**WO-A-82/03330**

**BIOLOGICAL ABSTRACTS, vol. 71, 1981, no.
56660; C. TREPO et al.: "Non-A, non-B
hepatitis: Identification of 2 antigen associated
with a hepatitis B-like virion and cross reacting
with hepatitis B core and hepatitis B e
antigens"**

(73) Proprietor: **Eisai Co., Ltd.
6-10, Koishikawa 4-chome Bunkyo-ku
Tokyo 112 (JP)**

(72) Inventor: **Tomatsu, Junichi
Warabi residence 511 4-1-5, Kitamachi
Warabi-shi Saitama (JP)**
Inventor: **Morimoto, Tomiaki
3-15-1, Shinmachi
Hino-shi Tokyo (JP)**
Inventor: **Shikata, Toshio
1-17-2, Motokitakata
Ichikawa-shi Chiba (JP)**

(74) Representative: **Hansen, Bernd, Dr.rer.nat. et al
Hoffmann, Eitle & Partner Patentanwälte
Arabellastrasse 4
D-8000 München 81 (DE)**

Courier Press, Leamington Spa, England.

## Description

This invention relates to a type non-A, non-B hepatitis-associated antigen and a diagnostic reagent utilizing such an antigen. More specifically, the present invention relates to a type non-A, non-B hepatitis-associated antigen, a conjugate of said antigen and a type non-A, non-B hepatitis diagnostic reagent containing said antigen or said conjugate of said antigen as its principal ingredient.

The existence of viral hepatitis of types A and B is known and an antigen-antibody system associated with these types of hepatitis has also been clarified. Immunoserological diagnosis using such systems has become possible and development and introduction of vaccines has been accomplished.

In the course of research, however, the existence of viral hepatitis which belongs neither to type A hepatitis nor to type B hepatitis, has become known and the frequency of occurrence thereof has been higher than expected. Type non-A, non-B hepatitis accounts for more than 80% up to 90% of post-transfusion hepatitis and it has been reported that type non-A, non-B hepatitis also accounts for about 50% of sporadic acute hepatitis. It has been established that even among normal blood donors, a considerable number of donors carry the non-A, non-B hepatitis virus. The following literature references set forth the state of the art pertaining to non-A, non-B hepatitis:

1) Prince, A.M. et al.: Long-incubation post-transfusion hepatitis without serological evidence of exposure to hepatitis-B virus. Lancet. II: 241—246, 1974
2) Alter, H.J. et al.: Clinical and serological analysis of transfusion-associated hepatitis. Lancet. II: 838—841, 1975.
3) Feinstone, S.M. et al.: Transfusion-associated hepatitis not due to viral hepatitis type A or B. N. Engl. J. Med. *292*: 767—770, 1975.
4) Meyers, J.D. et al.: Parenterally transmitted non-A, non-B hepatitis, Ann. Intern. Med. *87*: 57—59, 1977.
5) Villarejos, V.M. et al.: Evidence for viral hepatitis other than type A or type B among persons in Costa Rica. N. Engl. J. Med. *293:* 1350—1352, 1975.
6) Dienstag, J.L. et al.: Etiology of sporadic hepatitis B surface antigen-negative hepatitis. Ann. Intern. Med. *87*: 1—6, 1977.

The term "non-A, non-B hepatitis" used herein refers to those kinds of hepatitis which are believed to occur because of infection with virus(es) other than the previously known viruses, such as, type A hepatitis virus, type B hepatitis virus, cytomegalovirus and Epstein-Barr virus, which non-A, non-B hepatitis viruses participate in the disease as pathogens, and which do not exhibit a significant rise for HAV antibody, anti-HBs, anti-HBc and/or anti-HBe. Accordingly, non-A, non-B hepatitis patients can be defined as those who suffer from hepatitis resulting from infection by hepatitis viruses, but who are negative in both·the type A hepatitis diagnosis and the type B hepatitis diagnosis, and are therefore excluded from having either type A hepatitis or type B hepatitis. Generally, in comparison with type B hepatitis, the average incubation period from transfusion until outbreak, for non-A, non-B hepatitis, is shorter and the maximal value of glutamate - pyruvate - transaminase (GPT) is also lower. However non-A, non-B hepatitis has a longer duration so that the GPT abnormality, for example, becomes chronic and lasts longer. In any case, non-A, non-B hepatitis accounts for the major proportion of posttransfusion hepatitis. Liver diseases caused by the non-A, non-B hepatitis virus(es) include a variety of diseases such as liver cirrhosis and hepatocellular carcinoma. Accordingly, proper countermeasures must be devised as soon as possible. As to non-A, non-B hepatitis, however, the virus(es) that cause the disease have not yet been identified exactly and an antigen-antibody system associated therewith has not yet been established. Hence, proper diagnosis and vaccine production have not been achieved to date.

As prior art relating to a non-A, non-B hepatitis-associated antigen, mention can be made of Japanese Patent Laid-Open No. 122156/1980, which discloses a substance referred to as an "HC antigen" which is characterized in that it shows an antigen-antibody reaction exclusively with plasma or serum of a convalescent phase of type C hepatitis patients, but does not exhibit a generalized reactivity for non-A, non-B hepatitis. As the starting material for isolating and purifying the HC antigen, it is necessary to use plasma or serum taken from a group of patients suffering from the hepatitis, in the acute period of the illness, after transfusion of HBs negative blood, showing two or more peaks in the GPT rise and having a relatively long latent period. Hence, the foregoing prior invention is greatly restricted by its starting material.

WO application No. 82/03330 also describes NANB antigens. However, the physical characteristics of these NANB antigens differ from those of the antigen claimed in the present application, especially with respect to their molecular weight. Besides, the antigen according to the application is isolated from liver homogenate, whereas the antigen according to above citation is obtained from plasma.

In view of the prior art described above, the inventor of the present invention attempted to isolate, as a separate substance, a non-A, non-B hepatitis-associated antigen generally existing in and specific to non-A, non-B hepatitis, and succeeded in obtaining the contemplated substance by isolation and purification of autopsy liver from non-A, non-B hepatitis patients. Thus, the present invention has been completed on the basis of this discovery.

The substance of the present invention is isolated and purified from non-A, non-B hepatitis autopsy liver and has the following physicochemical properties. It has a molecular weight of at least 1,500,000 as determined by a gel filtration process. The sedimentation constant ($10^{-13}$) is 51.5S when measured with a Beckmann Model E ultra-centrifuge. The buoyant density ($g/cm^3$) in a cesium chloride solution or potassium bromide solution is 1.15 to 1.25 when measured with an Abbe's refractometer. The particle size (nm) ranges from 26 to 37 (31.5 on the average) under electron microscope observation. The electrophoretic mobility is in the $\alpha_2$—$\alpha_1$ globulin range when determined by immunoelectrophoresis in agarose gel.

As is obvious from these properties, the substance of the present invention is essentially different from the HC antigen disclosed in Japanese Patent Laid-Open No. 122156/1980 as to its starting material as well as in its physicochemical properties.

As will be illustrated in the Experimental Examples below, the substance of the present invention undergoes a precipitation reaction in an agarose gel with the serum of a convalescent phase from non-A, non-B hepatitis patients and with the serum of multiply-transfused hemophilia patients. Sheep erythrocytes sensitized by the substance of the present invention exhibit specific and highly sensitive passive hemagglutination with the serum of a convalescent phase from non-A, non-B hepatitis patients. On the other hand, the substance of the present invention does not react with HAV antibody, anti-HBs, anti-HBc, anti-HBe, anti-δ, HC antibody, EB antibody, Arai antibody, F antibody, normal human liver supernatant antibody and various normal human plasma antibodies. Accordingly, the substance of the present invention is a useful substance which is a specific antigen associated with non-A, non-B hepatitis and which is general to non-A, non-B hepatitis. As will be illustrated in Experimental Example 4, for instance, the substance of the invention is useful for providing accurate information in the course of diagnosis of non-A, non-B hepatitis.

An immunized antibody to the substance of the present invention can be produced by repeatedly immunizing rabbits with the substance of the invention together with a suitable adjuvant, such as Freund's complete adjuvant. For this reason, the substance of the present invention is expected to be useful as a starting material for producing non-A, non-B hepatitis vaccine.

The substance of the present invention can be obtained from hepatitis autopsy liver of non-A, non-B patients, as the starting material, by a suitable combination of conventional protein fractionation methods, such as density gradient centrifugation, salting out, electrophoresis, gel filtration, affinity chromatography, isoelectrofocusing, ultrafiltration and Cohn's fractionation. In a preferred embodiment of the invention, the autopsy liver homogenate supernatant is first subjected to column chromatography using Sephacryl S-200® and the resulting antigen positive fraction is subjected to ultrafiltration for the purpose of concentration. Then ultracentrifugation and dialysis are carried out. It is preferred to carry out ultracentrifugation once or several times by using successively both sucrose density gradient centrifugation and cesium chloride density gradient centrifugation. The purified matter thus obtained shows a clear precipitin line with serum of convalescent phase from a non-A, non-B hepatitis patients by agarose gel diffusion, and it is confirmed that the precipitin line completely fuses with a preciptin line in the autopsy liver homogenate supernatant before purification. In other words, it is thereby confirmed that the substance of the present invention can be isolated in accordance with the isolation purification method of the invention without losing its immunological properties.

A diagnostic reagent containing the substance of the present invention as its principal ingredient may then be suitably prepared in accordance with the immunological detection method to be employed. When a method such as micro-Ouchterlony's test (MO), immunoelectrophoresis (IES, IEP), complement fixation (CF) immune adherence hemagglutination (IAHA) or single radial immunodiffusion (SRID) is to be employed as the detection method, the substance of the present invention may be prepared as such in a suitable preparation. When, for example, the single radial immunodiffusion (SRID) is used, a suitable support is selected from agar, agarose, starch and polyacrylamide gel and the support is heat-dissolved in a buffer. After the substance of the present invention is added and mixed in, the resulting solution is flowed onto a glass plate or charged into a plastic container and then cooled to cause solidification. Finally, a hole for charging the serum to be tested is poured on the solidified gel plate.

However, when a method such as passive hemagglutination (PHA), radioimmunoassay (RIA) or enzyme-linked immunoadsorbent assay (ELISA) is used as the detection method, it is necessary to apply the corresponding suitable treatment to the substance of the present invention. For example, when passive hemagglutination is used, the substance of the present invention must be combined with fine carrier particles. The erythrocytes of mammals or birds are preferred as the fine carrier particles, but particles having a size of about 1 to 10 μm composed of polystyrene latex, polyester latex, vinyl chloride, bentonite or glass beads can also be used. The substance of the present invention can be combined with these fine particles by the use of glutaraldehyde, formaldehyde, tannic acid, bisdiazotized benzidine, chromium chloride or carbodiimide.

When the radioimmunoassay (RIA) method is used, the substance of the present invention must be labelled with an isotope. $^{125}I$ and $^{131}I$ can be used as the isotope and can be combined with the substance of the invention by the chloramine T method.

When the enzyme-linked immunoadsorbent assay (ELISA) method is used, the substance of the present invention must be combined with an enzyme. Examples of usable enzymes include glucose oxidase, alkali phosphatase, peroxidase and β-galactosidase. Glutaraldehyde can be used as the coupling agent.

The diagnostic reagent of the present invention is used for the diagnosis of non-A, non-B hepatitis, but it can be used, of course, as an experimental detection reagent or determination reagent for the non-A, non-B hepatitis antibody. Accordingly, the term "diagnostic reagent" in the present invention must not be interpreted as limiting the invention to diagnosis of non-A, non-B hepatitis.

Brief description of the drawings

Figure 1 is a schematic diagram showing the formation of the precipitin line when agarose gel diffusion was carried out according to micro-Ouchterlony's test in Experimental Example 1, and shows the arrangement of areas in which A represents the substance of the present invention, B is serum No. 1 of a convalescent phase from non-A, non-B hepatitis patients. C is serum No. 2 of such a patient, D is serum of a multiply-transfused hemophilia patients, E is normal human serum and F is normal human liver homogenate supernatant.

Figures 2 through 5 show clinical course, in respect to the non-A, non-B hepatitis antibody titer (line represented by o) and GPT (line represented by o) in serum from non-A, non-B hepatitis patients in Experimental Example 4.

Figure 6 is an electron micrograph showing the morphology of particles of the substance of the present invention.

Figure 7 is an electron micrograph showing the morphology of the antigen-antibody complex formed by the substance of the present invention.

The utility of the substance and the reagent of the present invention will be explained with reference to the following Experimental Examples.

Experimental Example 1
Samples:
A. Substance of the present invention, prepared in Example 1 below
B. A convalescent phase serum from non-A, non-B hepatitis patient, No. 1
C. A convalescent phase serum from non-A, non-B hepatitis patient, No. 2
D. Serum for multiply-transfused hemophilia patient
E. Normal human serum
F. Normal human liver homogenate supernatant

Method:
0.8% (w/v) of Agarose A-45 (Nakarai Kagaku), 0.02M EDTA · 3Na, 0.1M sodium chloride and 0.1% NaN$_3$ were dissolved in a Tris-HCl buffer (0.01M, pH 7.5) and a 1.5 mm-thick gel plate was prepared. A detection test was carried out for each sample A to F in accordance with the micro-Ouchterlony's test.

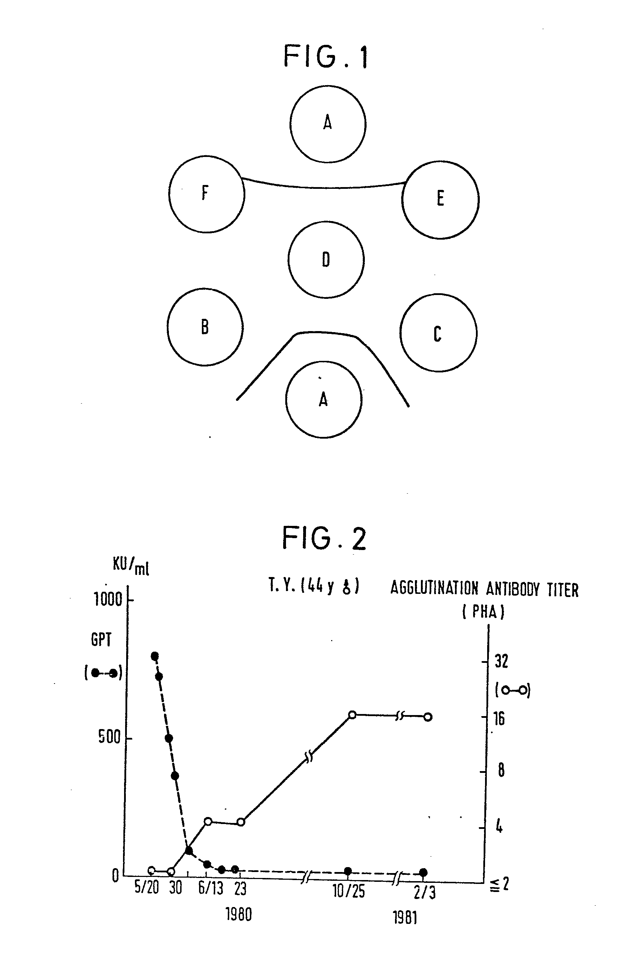

Results:
The result is shown in Figure 1. As is obvious from Figure 1, the substance of the present invention forms a precipitin line with both the convalescent phase serum from non-A, non-B hepatitis patient and with the serum from multiply-transfused hemophilia patient, and these precipitin lines completely fused with each other. According, it was found that they formed an antigen-antibody system having immunologically identity. This antigen-antibody system did not at all show a cross reaction with the normal human serum and with the normal human liver homogenate supernatant.

Experimental Example 2
Sample:
Sheep erythrocytes were fixed by a glutaraldehyde solution and treated with a tannic acid solution. Thereafter, they were sensitized by the substance of the present invention prepared in Example 1 below and the resulting sensitized blood cells were used as the blood cell sample using the substance of the present invention.

Method:
25 μl of a phosphate buffered saline (0.15M, pH 7.2) containing 2% normal rabbit serum was droped into a microtitration plate (V-plate) and the test serum was diluted by $2^n$ times in two series. 25 μl of a phosphate buffered saline (0.15M, pH 7.2) containing 2% normal rabbit serum was added to one of the series while 25 μl of the substance of the present invention was added to the other. After incubation at 37°C for 30 minutes, the sensitized blood cells were added to each well, and the plate was further incubated at room temperature for 2 hours so as to confirm the occurrence of agglutination. When the agglutination titer was three tubes (dilution of 8 times) or more for the buffer-containing samples and inhibition of agglutination due to the addition of the substance of the present invention was confirmed for at least two tubes, the solution was determined as being antibody positive and the titer was expressed as the highest dilution of the sample that showed hemagglutination.

Results:
The sensitivity of the hemagglutination reaction due to the antigen-sensitized blood cells prepared as described above was 100 to 200 times that of the agarose gel diffusion in Experimental Example 1. The specificity of the agglutination antibody could be clearly confirmed by the existence of agglutination inhibition due to both the present antigen and the normal human liver homogenate supernatant.

Experimental Example 3

Samples:

1. Sensitized blood cells described as the sample of Experimental Example 2; and
2. Test samples of the following four kinds (a) through (d):

(a) serum from 14 patients with acute viral hepatitis type A patients
(b) serum from 12 patients with viral hepatitis type B
(c) serum from 15 patients with acute viral hepatitis of non-A, non-B type
(d) serum of 129 normal donors

Method:

Detection frequency was confirmed in each subject by use of the passive hemagglutination reaction. Each blood serum was diluted $2^n$ times in two series. After the substance of the present invention (in the form of a solution diluted 50 times) was added to each sample of each of the series, the systems were incubated at 37°C for 30 minutes. The sensitized blood cells were added and two to three hours later the degree of agglutination was observed. The serum was determined as being antibody positive when an antibody titer of at least 3 tubes (dilution of 8 times) was observed and the inhibition effect of at least two tubes due to the substance of the present invention was observed. The antibody titer was expressed as the highest dilution of the sample that showed hemagglumination.

Results:

The results are shown in Table 1 below.

TABLE 1

| Subject | Number of cases | Antibody positive cases (%) | Average agglutination value ($2^n$) |
|---------|-----------------|-----------------------------|--------------------------------------|
| a | 14 | 2 (14.3) | 1.43±0.73 (N.S.) |
| b | 12 | 2 (16.7) | 1.50±0.96 (N.S.) |
| c | 15 | 11 (73.3) | 3.67±1.53 (p<0.001) |
| d | 129 | 9 (7.0) | 1.18±0.56 |

N.S.: no significant difference compared to (d)
p: level of significance
It was found from Table 1 that:

1. The percentage of positive cases was as high as 73.3% from the non-A, non-B hepatitis cases and a significant difference could be observed from the results for the normal donors, the hepatitis type A patients and the hepatitis type B patients.
2. The percentages of positive cases were 14.3% and 16.7% in the cases of hepatitis type A and type B, respectively, but no significant difference could be observed between these percentages and the percentages of positive cases for the normal donors.
3. The non-A, non-B hepatitis antibody was observed as existing in about 7% of the normal donors.
4. In the average agglutination values, no significant differences could be seen among blood serums of the normal donors, the hepatitis type A patients and the hepatitis type B patients, but the blood serum of the non-A, non-B hepatitis patients showed a significantly higher value (p 0.001) than the value of the normal donors.

From observations 1 through 4, it was concluded that the substance of the present invention is an antigen general for and specific to the non-A, non-B hepatitis antibody.

Experimental Example 4

Sample:

Serial serum of four patients having non-A, non-B hepatitis

Method:

The non-A, non-B hepatitis antibody level and GPT were measured for the serial serum. The non-A, non-B hepatitis antibody titer was measured using the substance of the present invention prepared in Example 1 below by means of the passive hemagglutination methods.

Results:

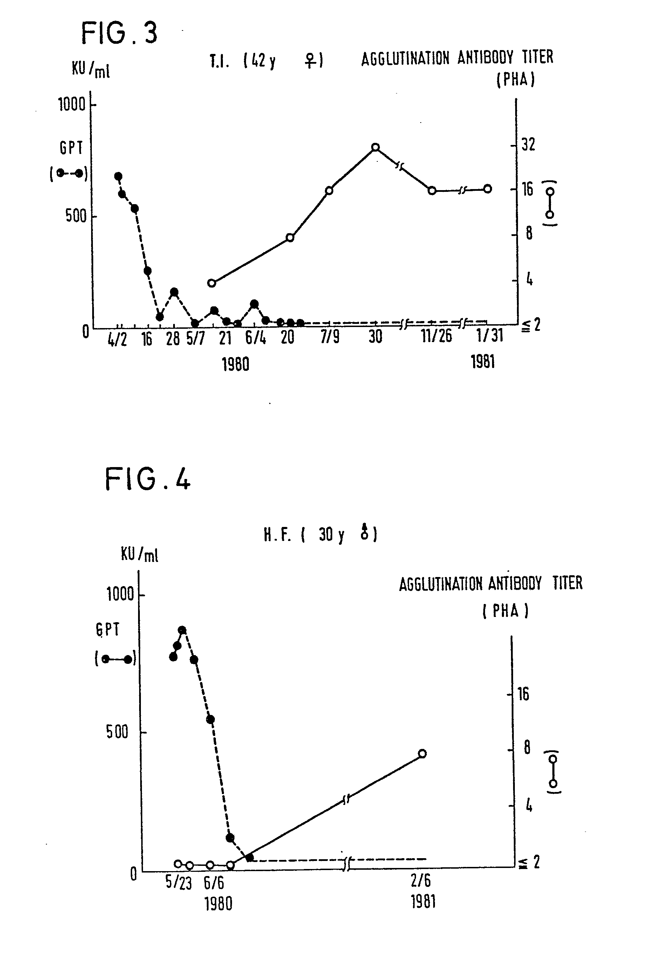

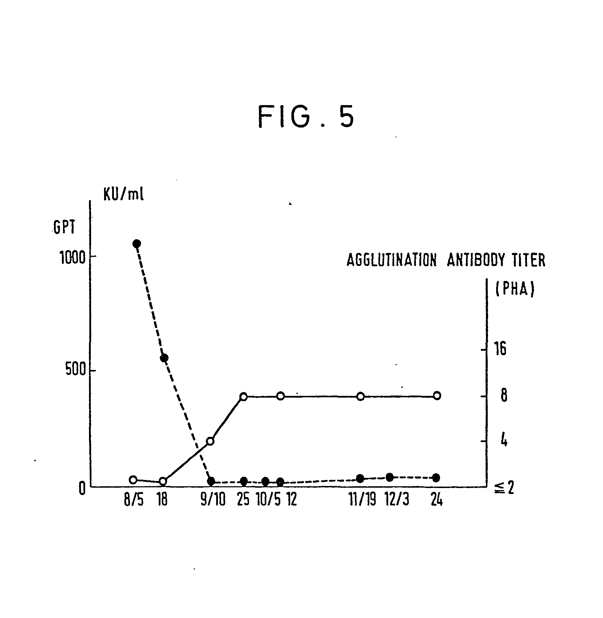

The results are shown in Figures 2 to 5. As is obvious from each figure, the non-A, non-B hepatitis antibody was not detected, or was detected only slightly, in the serum immediately after the outbreak (high point of the GPT curve) but it appeared in the blood serum along the normalization of the liver function (decrease of the GPT values). It was confirmed that the antibody titer remained for more than one year or two. Since the non-A, non-B hepatitis antibody was therefore found to serve as an in-blood marker useful for the diagnosis and prognostic observation of non-A, non-B hepatitis, the substance of the present invention which is general for and specific to said antibody provides an accurate diagnostic reagent for non-A, non-B hepatitis.

The following examples will further illustrate the present invention.

Example 1

About 5 g of the liver obtained on autopsy of a non-A, non-B hepatitis patient was sliced and homogenized by adding 20 ml of Tris-HCl buffer physiological salt solution (0.01M, pH 7.5). The solution was centrifuged at 9,000 g for 20 minutes and the homogenate supernatant was collected to obtain a liver homogenate supernatant for isolation and purification.

10 ml of the liver homogenate supernatant was charged into a column (2.6×90 cm) packed with Sephacryl S-200® (a product of Pharmacia) equilibrated in advance with Tris-HCl buffered saline (0.01M, pH 7.5), and was eluted by Tris-HCl buffer physiological salt solution (0.01M, pH 7.5). A fraction corresponding to the first peak having an antigen activity was collected by continuously monitoring the eluate with ultraviolet absorption at 280 nm. The antigen activity was determined by agar gel diffusion. The fraction thus collected was concentrated by ultrafiltration (PM-10®, a product of Amicon Corporation).

The resulting concentrate was subjected to elution in the same manner as described above but using Sephacryl S-300® (a product of Pharmacia) in place of the Sephacryl S-200®, and was again concentrated by ultrafiltration.

5 ml of the resulting concentrate was subjected to sucrose density gradient centrifugation 70,000 g, 16 hours). The density gradient ranged from 5 to 60% (W/V) and 10 ml each at 5%, 15%, 25% and 35% and 5 ml each at 45% and 60% were obtained. The concentrate was added to the uppermost portion. After the treatment, fractionation was carried out at a rate of 2.5 ml/tube from the tube bottom and a fraction corresponding to a sucrose density of 25 to 40% having an antigen activity was collected. The fraction thus collected was dialyzed by Tris-HCl buffered saline (0.01M, pH 7.5) and was concentrated by ultrafiltration (PM-10®, a product of Amicon Corporation).

Next, the resulting concentrate was subjected to cesium chloride equilibrated density gradient centrifugation (130,000 g, 20 hours). Samples of 4.5 ml each were prepared within the density gradient range of 1.00 to 1.50 g/cm³ and the concentrate was added to the uppermost portion. After the treatment, fractionation was effected at a rate of 0.2 ml/tube from the upper layer and a fraction corresponding to a cesium chloride density of 1.15 to 1.25 g/cm³ having an antigen activity was collected. The specific gravity of each fraction was measured with an Abbe's refractometer.

The physicochemical properties of the substance contained in the resulting solutions are tabulated below and the substance was used as the substance of the present invention in each of the aforementioned Experimental Examples 1, 2, 3, 4 and 5:

molecular weight: 1,500,000 or more (gel filtration)

sedimentation constant $(10^{-13})$: 51.5S (ultracentrifugal analysis)

buoyant density (g/cm³): 1.15—1.25 (in cesium chloride and in potassium bromide)

particle diameter (nm): 26—37

electrophoretic mobility: $\alpha_2$—$\alpha_1$ globulin range (in agarose gel)

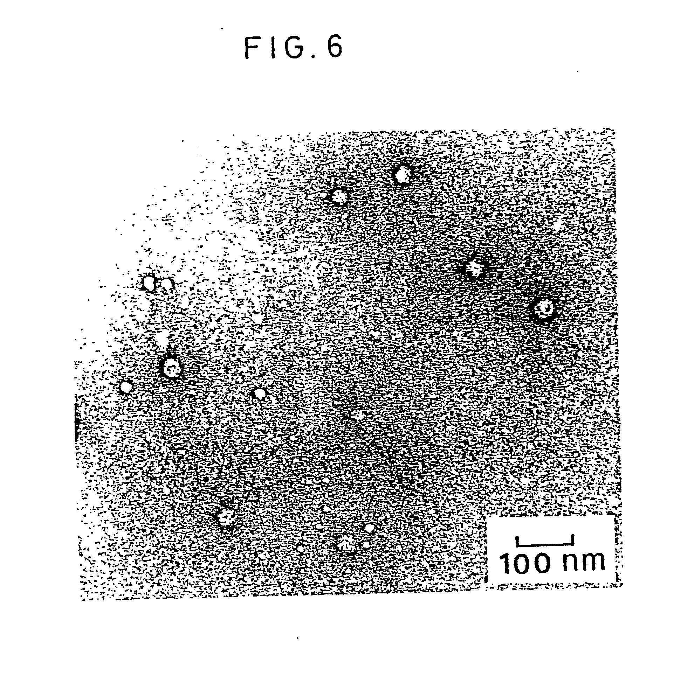

The electron micrograph of the substance of the present invention is shown in Figure 6.

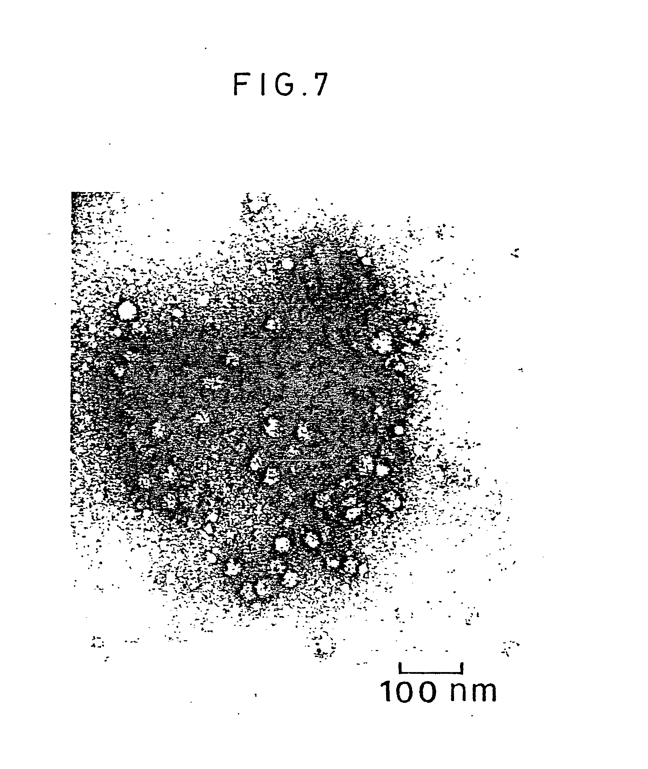

An antibody to the substance of the present invention was prepared from the non-A, non-B hepatitis, convalescent phase serum, mixed with the substance of the present invention in a test tube, incubated at 37°C for 60 minutes, left standing at 4°C over night, and thereafter centrifuged at 9,000 g for 20 minutes. As a result, the substance of the present invention was collected as a precipitate of an antigen-antibody complex. When the substance was observed by electron microscope, it was found that the spherical particles having a diameter of about 26 to 37 nm in Figure 6 agglutinated with one another. Figure 7 shows an image of an aggutination as a result of the antigen-antibody reaction.

Example 2

Freund's complete adjuvant was added to the solution obtained in Example 1 in the ratio of 1:1 and emulsified. 0.5 ml of the emulsion was injected in portions into the back of a rabbit, subcutaneously and intracutaneously, and to the foot pad of the rabbit, for immunization purposes. One week later, the same quantity of the emulsion was additionally injected in the same way and one month after the first immunization, a double quantity of the emulsion was further injected. One week after the first immunization, the blood of the rabbit was collected several times for separation of the blood serum. The same quantity of a normal human serum was added to this blood serum and the mixture was left standing at 37°C for one hour and thereafter at 4°C for one night. The mixture was centrifuged at 9,000 g for 10 minutes and the supernatant was collected. The antibody contained in the resulting anti-serum was purified by salting out using ammonium sulfate and column chromatography using DEAE cellulose, and then dissolved in a barbital buffer of 0.1M and pH 9.0 so as to adjust the protein concentration to 10 mg/ml. The solution was added to the same quantity of a cyanogen bromide-activated Sepharose 4B® gel and was reacted therewith at 4°C for 24 hours. Next, after the unreacted protein was removed by washing with a phosphate buffered saline of 0.05M and pH 7.5, the solution was charged into a 20 cm-long column having a 1.5 cm diameter. The solution obtained in Example 1 was added to this column. After the unreacted matter was removed by washing with the above-mentioned phosphate buffer physiological salt solution, an elutant was added to the column to effect elution. The eluate thus obtained was dialyzed with distilled water to remove the elutant, and concentrated by means of an Amicon Diaflow XM-50®. The solution obtained was used as a non-A, non-B hepatitis-associated antigen for producing a diagnostic reagent according to the PHA process, RIA, process and EIA process of Examples 3, 4 and 5 below.

Example 3

Sheep blood was charged into a centrifugal tube and centrifugation was repeated five times at 1,000 g for 10 minutes using a saline to wash the erythrocytes. A phosphate buffered saline of pH 7.5 and 0.15 M was added to the erythrocytes in a 5% concentration. One-fifth by volume of a glutaraldehyde solution prepared in a 2.5% concentration using the same phosphate buffered saline was added to a suspension of the above-mentioned erythrocytes, and the mixture was reacted with stirring at room temperature for about 5 hours to fix the erthrocytes. Next, this solution was centrifuged to obtain fixed erythrocytes, which were washed several times by centrifugation using the saline. These fixed erythrocytes were then prepared in the form of a 5% suspension using the above-mentioned phosphate buffer and the same quantity of a tannic acid solution prepared in 5 mg/dl using the same phosphate buffered saline was added to the solution. The mixed solution was then stirred for 30 minutes, and centrifuged to obtain tannic acid-treated and fixed erythrocytes, which were further washed several times by centrifugation using a saline. The phosphate buffer was added to the tannic acid-treated and fixed erythrocytes, thereby forming a 5% erythrocyte suspension.

The erythrocyte suspension was mixed with the same quantity of a solution of Example 2 in a protein concentration of about 10 µg/ml using the phosphate buffered saline, and was stirred at room temperature for 60 minutes to achieve sensitization. The solution was centrifuged to obtain sensitized blood cells, which were washed several times by centrifugation using the saline. A phosphate buffered saline containing 2% normal rabbit serum was added to the resulting sensitized blood cells to obtain a 7% blood cell suspension. This sensitized blood cell suspension was used as a diagnostic reagent according to the PHA method.

Example 4

The solution obtained in Examples 1 or 2 was dissolved in a phosphate buffer of pH 7.5 and 0.05M concentration to adjust the protein concentration to 1 mg/ml. 50 µl of $^{125}$I-Na of 1 mCi was added to 10 µl of this solution and 10 µl of a solution obtained by dissolving chloramine T in the above-mentioned phosphate buffer at a concentration of 1.5 mg/ml was further added. The mixed solution was stirred for 30 seconds, and 100 µl of a solution obtained by dissolving sodium metabisulfite in the above-mentioned phosphate buffer at a concentration of 2 mg/ml was added thereto to stop the reaction. 100 µl of a solution obtained by dissolving potassium iodide at a concentration of 10 mg/ml was added to this reaction solution and an $^{125}$I-labelled substance was immediately isolated from $^{125}$I by gel filtration using Sephadex G-50®. The specific activity of the resulting $^{125}$I-labelled substance was found to be about 20 to 60 µCi/µg. This $^{125}$I-labelled substance was used as a diagnostic reagent according to the RIA process.

Example 5

The solution obtained in Example 1 or 2 was concentrated to 5 to 10 mg/ml in 0.05M phosphate buffered saline. Alkali phosphatase (5 mg/ml) was added to 0.1 to 0.2 ml of this solution at a rate of 0.3 ml and the mixture was stirred. Next, 50 µm of 2.5% glutaraldehyde solution was added and the mixed solution was stirred at room temperature for 30 minutes. The solution was dialyzed over night using a 0.05M Tris-HCl buffer (pH 8.0) and then centrifuged at 1,000 g for 10 minutes. The supernatant substance was used as the enzyme-labelled antigen diagnostic reagent in the EIA process.

Example 6

Agarose was heat-dissolved in a Tris-HCl buffer of pH 7.5 and 0.01M (containing 0.15M of sodium chloride and 0.1% of sodium azide) to obtain a 1.2% agarose solution, and 80 ml of this solution was cooled down to about 56°C. 20 ml of the solution obtained in Example 1 was added to this solution and the mixed solution was stirred. The solution was then charged into a plastic container and left standing to form a 1 mm-thick agarose coated plate.

A plurality of sample holes having a 3 mm diameter were poured on this plate at a predetermined distance from each other.

The resulting material was used as a diagnostic reagent for use in the SRID process.

**Claims**

1. An antigen associated with non-A, non-B hepatitis obtained by isolation and purification from the liver of a mammal infected with non-A, non-B hepatitis, said antigen having the following physicochemical properties:

(a) molecular weight: 1,500,000 or more, by gel filtration;
(b) sedimentation constant $(10^{-13})$: 51.5S, by ultracentrifugal analysis;
(c) buoyant density $(g/cm^3)$: 1.15—1.25, in cesium chloride and in potassium bromide;
(d) particle diameter: approximately 26—37 nm; and
(e) electrophoretic mobility: $\alpha_2$—$\alpha_1$ globulin region, in agarose gel.

2. A conjugate suitable for use as a diagnostic reagent for detecting non-A, non-B hepatitis comprising the antigen of claim 1 combined with a substance selected from fine carrier particles suitable for passive hemagglutination procedure, labelling isotopes suitable for radioimmunoassay procedure, and enzymes suitable for enzyme-linked immunoadsorbent assay procedure.

3. A conjugate as claimed in Claim 2 wherein said fine carrier particles are sheep erythrocytes, said isotope is $^{125}I$ or $^{131}I$ and said enzyme is alkali phosphatase.

4. A non-A, non-B hepatitis diagnostic reagent containing as its principal ingredient the antigen of claim 1.

5. A non-A, non-B hepatitis diagnostic reagent containing, as its principal ingredient, the conjugate of claim 2.

6. A non-A, non-B hepatitis diagnostic reagent as claimed in Claim 5 wherein said fine particles are sheep erythrocytes, said isotope is $^{125}I$ or $^{131}I$ and said enzyme is alkali phosphatase.

7. An antigen according to Claim 1, wherein said antigen is isolated and purified by:

(a) slicing and homogenizing said autopsy liver;
(b) centrifuging the homogenized autopsy liver to form a liver homogenate supernatant;
(c) subjecting said supernatant homogenate to column chromatography one or more times to isolate an antigen positive fraction; and
(d) subjecting said antigen positive fraction to ultracentrifugation one or more times to obtain said antigen.

8. An antigen according to Claim 7, wherein in said step (c) said homogenate supernatant is eluted through an equilibrated column packed with Sephacryl S200® a copolymer of N,N'-methylene-bisacrylamide and allyldextrane, concentrated, then again eluted through an equilibrated column packed with Sephacryl S-300® a copolymer of N,N'-methylene-bisacrylamide and allyldextrane and then concentrated a second time; and in step (d) said antigen positive fraction is subjected successively to sucrose density gradient centrifugation and then cesium chloride density gradient centrifugation.

9. An antigen according to Claim 1, Claim 7 or Claim 8 which forms a precipitin line with the serum of a convalescent phase from non-A, non-B hepatitis patient in the micro-Ouchterlony's method, said antigen being non-reactive with HA antibody, HBs antibody, HBe antibody, HBc antibody, δ antibody, HC antibody, EB antibody, Arai antibody, F antibody, normal human liver homogenate supernatant antibody and various normal human plasma antibodies.

10. An antibody to the antigen according to Claims 1 to 9, wherein said antibody is obtained by a process comprising the steps of immunizing rabbits with said antigen together with Freund's complete adjuvant, thereby causing formation of an antiserum; and collecting said antiserum from said animal and subjecting said antiserum to centrifugation at 9,000 g to obtain said antibody in the supernatant.

**Patentansprüche**

1. Nicht-A, nicht-B-Hepatitis-assoziiertes Antigen, das erhalten wird durch Isolierung und Reinigung aus der Leber eines Säugers, die nicht-A, nicht-B-Hepatitis-infiziert ist, wobei das genannte Antigen die folgenden physikalischchemischen Eigenschaften aufweist:

(a) Molekulargewicht: 1.500.000 oder darüber (Gelfiltration);
(b) Sedimentationskonstante $(10^{-13})$: 51,5S (analytische Ultrazentrifugation);
(c) Auftriebsdichte $(g/m^3)$: 1,15—1,25, in Cäsiumchlorid und in Kaliumbromid;
(d) Teilchendurchmesser: umgefähr 26—37 nm; und
(e) elektrophoretische Mobilität $\alpha_1—\alpha_2$-Globulin-bereich in Agarosegel.

2. Konjugat als diagnostisches Reagens zum Nachweis von nicht-A, nicht-B-Hepatitis, gekennzeichnet, durch das Antigen von Anspruch 1 in Kombination mit einer Substanz, die ausgewählt ist aus feinen Trägerteilchen, die für das passive Hämaglutinationsverfahren geeignet sind, Markerisotope, die sich für die Radioimmunobestimmung eignen, und Enzyme, die geeignet sind für das Enzym-gebundene Immunoadsorbens-Bestimmungsverfahren.

3. Konjugat nach Anspruch 2, dadurch gekennzeichnet, dass die feinen Trägerteilchen Schaf-Erythrocyten, das genannte Isotop $^{125}I$ oder $^{131}I$ und das genannte Enzym alkalische Phosphatase darstellen.

4. Diagnostisches Reagens auf nicht-A, nicht-B-Hepatitis, welches als Hauptbestandteil das Antigen gemäss Anspruch 1 enthält.

5. Diagnostisches Reagens auf nicht-A, nicht-B-Hepatitis, welches als Hauptbestandteil das Konjugat gemäss Anspruch 2, enthält.

6. Diagnostisches Reagens auf nicht-A, nicht-B-Hepatitis gemäss Anspruch 5, dadurch gekennzeichnet, dass die genannten feinen Teilchen Schaf-Erythrocyten das genannte Isotop $^{125}I$ oder $^{131}I$ und das genannte Enzym alkalische Phosphatase darstellen.

7. Antigen nach Anspruch 1, dadurch gekennzeichnet, dass das Antigen wie folgt isoliert und gereinigt wird:

(a) Aufschneiden und Homogenisieren von durch Autopsie erhaltener Leber;
(b) Zentrifugieren der durch Autopsie erhaltenen Leber unter Bildung eines Leberhomogenat-Überstandes;
(c) ein- oder mehrmalige säulenchromato-grafische Behandlung des Homogenat-Über-standes zur Isolierung einer Antigen-positiven Fraktion; und
(d) ein- oder mehrmaliges Ultrazentrifugieren der genannten Antigen-positiven Fraktion zum Erhalt des genannten Antigens.

8. Antigen nach Anspruch 7, dadurch gekennzeichnet, dass in dem genannten Schritt (c) der Homogenat-Überstand von einer equilibrierten Säule eluiert wird, welche mit Sephacryl S-200®, einem Copolymer von N,N'-Methylen-bisacrylamid und Allyldextran, gefüllt ist, daraufhin konzentriert wird und dann wiederum von einer equilibrierten Säule eluiert wird, die mit Sephacryl S-300®, einem Copolymer aus N,N'-Methylen-bisacrylamid und Allyldextran, gefüllt ist, und dann ein zweites Mal konzentriert wird; und in Schritt (d) die genannte Antigen-positive Fraktion nacheinander einer Zentrifugation in einem Sucrose-Dichtegradienten und dann einer Zentrifugation in einem Cäsiumchlorid-Dichtegradienten unterworfen wird.

9. Antigen nach Anspruch 1, 7 oder 8, welches mit den Serum eines sich in der Rekonvaleszenzphase befindenden nicht-A, nicht-B-Heptitis-Patienten beim Mikro-Ouchterlony-Verfahren eine Präzipitinlinie bildet, wobei das genannte Antigen mit HA-Antikörper, HBs-Antikörper, HBe-Antikörper, HBc-Antikörper, δ-Antikörper, HC-Antikörper, EB-Antikörper, Arai-Antikörper, F-Antikörper, einem Antikörper im Überstand von normalen menschlichen Leberhomogenat und verschiedenen Antikörpern im normalen menschlichen Plasma nicht reagiert.

10. Antikörper auf das Antigen gemäss Anspruch 1 bis 9, dadurch gekennzeichnet, dass der genannte Antikörper durch ein Verfahren erhalten wird, das gekennzeichnet, ist durch Schritte zum Immunisieren von Kaninchen mit dem genannten Antigen zusammen mit Freund's komplettem Adjuvans, wodurch die Bildung eines Antiserums veranlasst wird; und Sammeln des Antiserums von dem genannten Tier, und Zentrifugation des genannten Antiserums bei 9.000 g zum Erhalt des entsprechenden Antikörpers im Überstand.

**Revendications**

1. Antigène associé à l'hépatite non-A, non-B, obtenu par isolement et purification à partir du foie d'un mammifère atteint d'une hépatite non-A, non-B, l'antigène ayant les propriétés physico-chimiques suivantes:

(a) masse moléculaire: 1 500 000 ou plus, par filtration sur gel;
(b) constante de sédimentation $(10^{-13})$: 51,5 S, par analyse par ultracentrifugation;
(c) densité apparente $(g/cm^3)$: 1,15—1,25 dans du chlorure de césium et dans du bromure de potassium;
(d) diamètre des particules: environ 26 à 37 nm; et
(e) mobilité électrophorétique: dans la gamme de la $a_2$—$\alpha_1$ globuline, dans un gel d'agarose.

2. Conjugué approprié pour l'utilisation comme réactif diagnostique pour la détection de l'hépatite non-A, non-B, comprenant l'antigène selon la revendication 1 combiné avec une substance choisie parmi de fines particules de support utilisables pour une opération d'hémagglutination passive, des isotopes radioactifs convenant pour un dosage radio-immunologique et des enzymes appropriées pour un dosage d'immunoadsorbant fixé sur une enzyme.

3. Conjugué selon la revendication 2, dans lequel les fines particules de support sont des érythrocytes de mouton, l'isotope est $^{125}$I ou $^{131}$I et l'enzyme est une phosphatase alcaline.

4. Réactif diagnostique de l'hépatite non-A, non-B, contenant comme principal ingrédient l'antigène selon la revendication 1.

5. Réactif diagnostique de l'hépatite non-A, non-B, contenant comme principal ingrédient le conjugué selon la revendication 2.

6. Réactif diagnostique de l'hépatite non-A, non-B, selon la revendication 5, dans lequel les fines particules sont des érythrocytes de mouton l'isotope est $^{125}$I ou $^{131}$I et l'enzyme est une phosphate alcaline.

7. Antigène selon la revendication 1, dans lequel l'antigène est isolé et purifié par:

(a) découpage en tranches et homogénéisation du foie autopsié;
(b) centrifugation du foie autopsié homogénéisé de manière à former une substance surnageante d'homogénéisat de foie;
(c) chromatographie sur colonne de l'homogénéisat surnageant, une ou plusieurs fois, pour isoler une fraction antigène-positive; et
(d) ultracentrifugation de la fraction antigène-positive, une ou plusieurs fois, pour obtenir l'antigène.

8. Antigène selon la revendication 7, dans lequel au stade (c) on élue la substance surnageante d'homogénéisat à travers une colonne équilibrée, garnie avec du Sephacryl S-200®, un copolymère de N,N'-méthylène-bisacrylamide et d'allyldextrane, on concentre, puis on élue de nouveau sur une colonne équilibrée garnie avec du Sephacryl S-300®, un copolymère de N,N'-méthylène-bisacrylamide et d'allyldextrane, puis on concentre une seconde fois; et au stade (d) on soumet la fraction antigène-positive à une centrifugation avec gradient de densité de saccharose et à une centrifugation avec gradient de densité de chlorure de césium.

9. Antigène selon la revendication 1, 7 ou 8, qui forme une ligne de précipitine avec le sérum d'une phase convalescente d'un patient atteint d'hépatite non-A, non-B, dans le micro-procédé d'Ouchterlony, l'antigène ne réagissant pas avec l'anticorps HA, l'anticorps HBs, l'anticorps HBe, l'anticorps HBc, l'anticorps δ, l'anticorps HC, l'anticorps EB, l'anticorps Arai, l'anticorps F, l'anticorps de la substance surnageante d'homogénéisat de foie humain normal, ni avec divers anticorps de plasma humain normal.

10. Anticorps opposé à l'antigène selon les revendications 1 à 9, dans lequel l'anticorps est obtenu par un procédé comprenant les opérations qui consistent à immuniser des lapins avec l'antigène en même temps qu'avec l'adjuvant complet de Freund, de manière à entraîner la formation d'un antisérum; puis à recueillir l'antisérum de l'animal et à soumettre cet antisérum à une centrifugation à 9 000 g pour obtenir l'anticorps dans la substance surnageante.

0 066 296

# FIG. 1

# FIG. 2

1

# FIG. 3

KU /ml

T.I. ( 42 y ♀ )     AGGLUTINATION ANTIBODY TITER
(PHA)

# FIG. 4

H.F. ( 30 y ♂ )

FIG . 5

3

# FIG. 6

100 nm

FIG.7

100 nm